(19) Europäisches Patentamt
European Patent Office
Office européen des brevets

(11) EP 2 076 769 B1

(12) **EUROPEAN PATENT SPECIFICATION**

(45) Date of publication and mention
of the grant of the patent:
**25.04.2018 Bulletin 2018/17**

(21) Application number: **07817819.1**

(22) Date of filing: **02.10.2007**

(51) Int Cl.:
*G01N 33/50* (2006.01)     *C07K 16/28* (2006.01)

(86) International application number:
**PCT/DK2007/000421**

(87) International publication number:
**WO 2008/040348 (10.04.2008 Gazette 2008/15)**

(54) **HIGH THROUGPUT METHODS FOR CHARACTERIZATION OF ANTIBODIES**

VERFAHREN MIT HOHEM DURCHSATZ ZUR CHARAKTERISIERUNG VON ANTIKÖRPERN

PROCÉDÉS HAUT DÉBIT DE CARACTÉRISATION D'ANTICORPS

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **03.10.2006 DK 200601281**
**28.06.2007 DK 200700937**

(43) Date of publication of application:
**08.07.2009 Bulletin 2009/28**

(73) Proprietor: **Genmab A/S**
**1560 Copenhagen V (DK)**

(72) Inventors:
• **GERRITSEN, Arnout, F.**
**3981 TB Bunnik (NL)**
• **PARREN, Paul**
**3984 PR Odijk (NL)**
• **BOSCH, Martijn**
**4702 PP Bergentheim (NL)**
• **POUCKE, Adinda van**
**4702 PP Roosendaal (NL)**
• **HOUTKAMP, Mischa**
**3991 JG Houten (NL)**

(74) Representative: **Genmab A/S**
**Kalvebod Brygge 43**
**1560 Copenhagen V (DK)**

(56) References cited:
WO-A-2006/055371     WO-A-2006/099875
US-A1- 2002 182 643     US-A1- 2004 018 198

• LEE-MACARY ALICE E ET AL: "Development of a novel flow cytometric cell-mediated cytotoxicity assay using the fluorophores PKH-26 and TO-PRO-3 iodide" JOURNAL OF IMMUNOLOGICAL METHODS, vol. 252, no. 1-2, 1 June 2001 (2001-06-01), pages 83-92, XP002479005 ISSN: 0022-1759
• WILKINSON R W ET AL: "Antibody-dependent cell-mediated cytotoxicity: a flow cytometry-based assay using fluorophores" JOURNAL OF IMMUNOLOGICAL METHODS, ELSEVIER SCIENCE PUBLISHERS B.V.,AMSTERDAM, NL, vol. 258, no. 1-2, 1 December 2001 (2001-12-01), pages 183-191, XP004311928 ISSN: 0022-1759
• MARTIN ROBERT M ET AL: "DNA labeling in living cells" CYTOMETRY. PART A, JOHN WILEY, HOBOKEN, NJ, US, vol. 67, no. 1, 1 September 2005 (2005-09-01), pages 45-52, XP009092963 ISSN: 1552-4922
• DIAZ TOMAS M ET AL: "FDA/PI flow cytometry assay of complement-mediated cytotoxicity of antibodies generated during xenotransplantation" CYTOMETRY, vol. 62A, no. 1, November 2004 (2004-11), pages 54-60, XP002479006 ISSN: 0196-4763

**Description**

**FIELD OF THE INVENTION**

The present invention relates to methods for the characterization of monoclonal antibodies, in particular high throughput methods for the characterization of antibodies with respect to internalization.

**BACKGROUND OF THE INVENTION**

Targeted therapies using monoclonal antibodies have achieved important therapeutic applications in the treatment of various human diseases, including cancer and autoimmune diseases.

Therapeutic effects of antibodies can be influenced by internalization of the antibody into target cells. Internalization may reduce the potential for a therapeutic antibody to induce tumor lysis by Fc-mediated effector function or may affect antibody pharmacokinetics, which may or may not be desirable depending on the intended use. Internalization and down-regulation of receptors may also be considered a favorable characteristic of antibodies for some therapeutic applications. Receptor down modulation may reduce cell activation or may reduce the ability of cells to become or remain activated by autologous or exogenous stimuli. Internalization capabilities of therapeutic antibodies may also be exploited for toxin-based treatment for cancers. By targeting the molecule of interest to the tumor by an antibody-toxin complex, tumors can be killed specifically via internalization of the complex. As internalization may be desirable in some instances and less desirable in others, it is of interest to study antibody internalization in early drug discovery.

Assays for the internalization of G-protein-coupled receptors using an antibody as a tool for detection have been described by Amersham Biosciences (website, posters 125 and 133). These assays use antibodies labeled with Cypher5, a pH-sensitive dye, to detect internalization of the G-protein-coupled receptors into the low pH endosome. Detection is performed by high-content subcellular analysis on an IN Cell Analyzer 3000 or 1000 and data analysis is done with algorithms using additional staining of the cells to determine the cell shape and cytoplasm. However, no high-throughput method for selection of internalized antibodies was described.

US 6,794,128 describes a method of selecting antibodies that are internalized from a phage display library by recovering infectious phage particles from within cells.

US 7,045,283 describes a method of screening for internalizing antibodies using a reporter that is non-covalently bound to the antibody. In this method, non-internalized reporter is dissociated from the cells thus allowing specific detection of reporter molecules that have internalized into cells.

The two above-described prior art methods for screening for internalizing antibodies are limited to screening of phage display libraries and/or require a reporter dissociation step, e.g. a washing step. Thus, there is still a need for convenient, more generally applicable, screening methods for antibody internalization.

US2004/018198 describes an assay for monitoring internalization of an antibody, wherein the test antibody is reacted with a fluorescently labelled (AlexaFluor546) secondary antibody. US2004/018198 does not disclose the use of a Fab fragment as a detection tool.

Therapeutic effects can also be influenced by the ability of an antibody to mediate complement-dependent cytotoxicity (CDC). E.g. IgG antibodies can, on binding to an antigen, activate complement, such as via the classical pathway of complement activation, leading to lysis of the cell on which the antigen is located and/or to attraction of immune cells (chemotaxis) which phagocytose the antigen-expressing cells. Thus, it is of interest to study the ability of antibodies to activate CDC in early discovery.

CDC is often measured using fluorescent dyes, such as Alamar Blue (see e.g. Lazar et al. (2006) Proc. Natl. Acad. Sci. USA103, 4005-4010) or using tetrazolium bromide (MTT) (see e.g. Brezicka et al. (2000) Cancer Immunol. Immunother. 49:235-242). It is also possible to perform CDC assays with chromium labeled cells (Paget et al. (1978) Arthritis Rheum. 21:249-255). However, many CDC assays are not suitable for high throughput, require normalization, are not accurate and/or require high antibody concentrations. Thus, there is still a need for convenient, accurate, screening methods for antibody-mediated CDC.

Antibody-dependent cellular cytotoxicity (ADCC) represents a further mechanism through which antibodies may exercise therapeutic effects. A variety of effector cells, including NK cells, PMNs, and monocytes/macrophages, have receptors for the Fc region of antibodies, through which they can mediate killing of target cells, such as tumor cells. This often involves release of toxic molecules at the surface of the target cell. For antibodies that are to be used in cancer therapy, it is often desired that they can mediate ADCC. Thus, it is also of interest to study the ability of antibodies to activate ADCC in early stages of drug discovery.

A number of techniques have been devised for monitoring ADCC towards target cells. An often used method relies on chromium 51 labeling of target cells and measuring the release of chromium upon cytolysis (Brunner et al. (1968) Immunology 14: 181). Besides the obvious disadvantages of the use of a radioactive label, the chromium 51 assay may diffuse out of the cell, leading to an increase in background over time. This is inconvenient in a high throughput step-up. In addition, most known ADCC assays are end-point assays which provide a "snapshot" of the ADCC. Thus, technologies or methods that are suitable for a high throughput format and can provide kinetic information about ADCC are needed.

## SUMMARY OF THE INVENTION

The present invention provides a number of characterization assays for antibodies that may be used to select, from a plurality of antibodies, an antibody that exhibits characteristics that are desired for its contemplated use, e.g. therapeutic, prophylactic or diagnostic use.

In a first main aspect, the invention provides a method for selecting an antibody on the basis of its ability to be internalized into a cell, said method comprising the steps of

> a) providing a plurality of antibodies to be tested for internalization, wherein each of said antibodies is provided as a separate sample, and wherein said antibodies bind the same antigen,
> b) providing eukaryotic cells expressing the antigen to which said antibodies bind,
> c) providing a Fab fragment capable of binding said antibodies, wherein said Fab fragment i) does not mediate internalization, and ii) comprises a fluorescent label,
> d) incubating said samples, cells and Fab fragment in a test vial under conditions that allow formation and internalization of a complex comprising said antigen, antibody and Fab fragment,
> e) detecting fluorescence of at least a cell-containing subvolume of said test vial, and
> f) selecting an antibody on the basis of the outcome of the detection.

An antibody can be selected in step f) because it internalizes or not, depending on whether or not internalization is desired during the intended use of the antibody.

The above method of the invention provides a number of advantages over the internalization assays known in the art.

Firstly, separate direct labeling of each antibody to be tested is avoided by the use of a detection tool that recognizes any antibody, but does not mediate internalization itself.

Furthermore, the assay can be performed as a homogenous assay, i.e. it does not require time-consuming and/or laborious physical separation or washing steps. Thus, the assays are rapid, and can be performed in high throughput at relatively low cost.

Moreover, in one embodiment, the assay can be performed directly on crude supernatant samples from hybridoma or transfectoma cultures, thus avoiding laborious concentration and/or purification steps. Generally, the assay is highly sensitive, and thus allows analysis of samples that contain only low concentrations of antibody.

In addition, as the internalized labeled antibodies continue to accumulate intracellularly over time during the incubation in step d), it is possible to perform quantitative measurements to obtain kinetic data. Furthermore, by prolonged incubation, it is even possible to detect internalizing antibodies that recognize a target antigen that is only transiently exposed on the cell surface.

In its broadest aspect, the detection performed in step e) can be performed using any type of fluorescent detection method known in the art. In some embodiments, detection is performed using a macro-confocal laser scanner. The use of macro-confocal detection allows removal of background noise while retaining detection speed and no simultaneous nuclear staining is required.

In one embodiment, the label used is a dye that generates a different signal when it enters an intracellular compartment, such as an endosome or lysosome. This is advantageous, because such a dye can generate highly reliable data on internalization even in cells which have a high nucleus to cytoplasm ratio, and thus little cytoplasm relative to their size (e.g. lymphocytes). The use of this type of dye also further reduces background signal.

Also disclosed herein is a method for selecting an antibody on the basis of its ability to mediate complement-dependent cytotoxicity, said method comprising the steps of

> a) providing a plurality of antibodies to be tested for their capacity to mediate complement-dependent cytotoxicity, wherein each of said antibodies is provided as a separate sample, and wherein said antibodies bind the same antigen,
> b) providing target cells expressing the antigen to which said antibodies bind,
> c) providing complement,
> d) incubating said sample, target cells and complement under conditions that allow complement activation and target cell lysis,
> e) adding a first and a second dye, wherein the first dye is an anthraquinone or derivative thereof, and the second dye is a cyanine dye composed of a quinoline and a benzothiazole moiety, and
> f) performing count measurements of individual cells, wherein the reading discriminates between cells that are stained with both the first and the second dye, and cells that are only stained with the first dye, wherein the measurement is performed using a macro-confocal laser scanner,

g) calculating the relative proportion of dead cells, and

h) selecting an antibody on the basis of the outcome of the calculation.

The above method is also referred to as "the CDC method" herein.

Further disclosed herein is a method for selecting an antibody on the basis of its ability to mediate antibody-dependent cellular cytotoxicity, said method comprising the steps of

a) providing a plurality of antibodies to be tested for their capacity to mediate antibody-dependent cellular cytotoxicity, wherein each of said antibodies is provided as a separate sample, and wherein said antibodies bind the same antigen,

b) providing target cells expressing the antigen to which said antibodies bind, wherein said target cells have been stained by pre-incubation with a first dye, wherein said first dye is an anthraquinone or derivative thereof,

c) providing effector cells,

d) incubating said sample, target cells and effector cells under conditions that allow effector cell activation and target cell lysis,

e) adding a second dye, wherein said second dye is a cyanine dye composed of a quinoline and a benzothiazole moiety,

f) performing count measurements of individual cells, wherein the reading discriminates between cells that are stained with both the first and the second dye, and cells that are only stained with the first dye, wherein the measurement is performed using a macro-confocal laser scanner,

g) calculating the relative proportion of dead cells, and

h) selecting an antibody on the basis of the outcome of the calculation.

This method is also referred to as "the ADCC method" herein.

The disclosed methods provide a number of advantages over the CDC and ADCC assays known in the art.

Firstly, the assays can be performed as homogenous assay, i.e. they do not require time-consuming and/or laborious physical separation or washing steps. Thus, the assays are rapid, and can be performed in high throughput at relatively low cost.

Furthermore, assays are highly sensitive, and thus allow analysis of samples that contain low concentrations of antibody. Thus, in one embodiment, the assay can be performed directly on crude supernatant samples from hybridoma or transfectoma cultures, thus avoiding laborious concentration and/or purification steps.

Moreover, a high degree of accuracy is obtained, because the use of a double stain and because all nuclei containing dye are counted as separate events.

In a further aspect, the invention relates to a method for selecting an antibody suitable for the treatment of disease, comprising performing, on a plurality of samples, the internalization method as described herein.

## BRIEF DESCRIPTION OF THE FIGURES

Figure 1: Internalization study on overnight 37°C cultured CHO-CD38 cells using antibody HuMabCD38-049 and Fab GtaHuIgG-cypHer5. HuMab-KLH and incubation at 4°C were used as negative control. A 50 counts cut off was used.

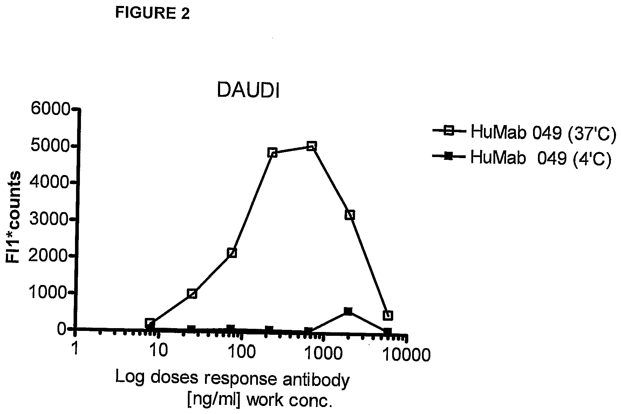

Figure 2: Internalization study on overnight cultured Daudi cells using antibody HuMabCD38-049 and Fab GtaHuIgG-cypHer5. Incubation at 4°C was used as negative control. No count cut off was used.

Figure 3: Internalization study on CHO-CD38 cells using HuMabCD38-049 and Fab GtaHuIgG-cypHer5 at room temperature. HuMab-KLH was used as negative control. A 100 counts cut off was used.

Figure 4: This figure shows the dose-response curves of CDC activity induced by different human antibodies on Daudi cells. The activity was measured using a dual stain of DRAQ5 and TOPRO-3.

Figure 5: This figure shows the dose-response curves of CDC activity induced by different human antibodies on CHO-CD38 cells. The activity was measured using a dual stain of DRAQ5 and TOPRO-3.

Figure 6: This figure shows the dose response curves of CDC activity induced by rituximab on Daudi cells. After completion of the CDC by stopping the reaction with the Azide buffer the plate was measured at different time points. The activity was measured using a dual stain of DRAQ5 and TOPRO-3.

Figure 7: This figure shows dose-response curves of CDC activity induced by rituximab and HuMab-KLH (negative control) on Daudi cells. The activity was measured using a dual stain of DRAQ5 and TOPRO-3.

Figure 8: This figure shows internationalization of anti-CD38 antibodies of cross-block group 1 in CHO-CD38 cells.

Figure 9: This figure shows internalization of anti-CD38 antibodies of cross-block group 2 in CHO-CD38 cells.
Figure 10: This figure shows internalization of anti-CD38 antibodies of cross-block group 3 in CHO-CD38 cells.
Figure 11: This figure shows internalization of anti-CD38 antibodies of cross-block group 4 in CHO-CD38 cells.
Figure 12: This figure shows mean fluorescent intensities of each of the four cross-block groups.

## DETAILED DESCRIPTION OF THE INVENTION

Definitions

The term "antibody" as used herein refers to an immunoglobulin molecule, a fragment of an immunoglobulin molecule, or a derivative of either thereof, which has the ability to specifically bind to an antigen under typical physiological conditions for significant periods of time, such as a time sufficient to induce, promote, enhance, and/or modulate a physiological response associated with antibody binding to the antigen and/or a time sufficient for the antibody to recruit an Fc-mediated effector activity.

The variable regions of the heavy and light chains of the immunoglobulin molecule contain a binding domain that interacts with an antigen. The constant regions of the antibodies may mediate the binding of the immunoglobulin to host tissues or factors, including various cells of the immune system (such as effector cells) and components of the complement system such as C1q, the first component in the classical pathway of complement activation.

As indicated above, the term "antibody" as used herein, unless otherwise stated or clearly contradicted by the context, includes fragments of an antibody that retain the ability to specifically bind to an antigen. It has been shown that the antigen-binding function of an antibody may be performed by fragments of a full-length antibody. Examples of binding fragments encompassed within the term "antibody" include (i) a Fab fragment, a monovalent fragment consisting of the $V_L$, $V_H$, $C_L$ and $C_H1$ domains; (ii) $F(ab)_2$ and $F(ab')_2$ fragments, bivalent fragments comprising two Fab fragments linked by a disulfide bridge at the hinge region; (iii) a Fd fragment consisting essentially of the $V_H$ and $C_H1$ domains; (iv) a Fv fragment consisting essentially of the $V_L$ and $V_H$ domains of a single arm of an antibody, (v) a dAb fragment (Ward et al., Nature 341, 544-546 (1989)), which consists essentially of a $V_H$ domain and also called domain antibodies (Holt et al. (November 2003) Trends Biotechnol. 21(11):484-90); (vi) camelid or nanobodies (Revets et al. (January 2005) Expert Opin Biol Ther. 5(1):111-24).and (vii) an isolated complementarity determining region (CDR). Furthermore, although the two domains of the Fv fragment, $V_L$ and $V_H$, are coded for by separate genes, they may be joined, using recombinant methods, by a synthetic linker that enables them to be made as a single protein chain in which the $V_L$ and $V_H$ regions pair to form monovalent molecules (known as single chain antibodies or single chain Fv (scFv), see for instance Bird et al., Science 242, 423-426 (1988) and Huston et al., PNAS USA 85, 5879-5883 (1988)). Such single chain antibodies are encompassed within the term antibody unless otherwise noted or clearly indicated by the context.

The term "antibody" also includes bispecific antibodies, diabodies, or similar molecules (see for instance PNAS USA 90(14), 6444-8 (1993) for a description of diabodies).

It should be understood that the term antibody also generally includes polyclonal antibodies, monoclonal antibodies, e.g. human antibodies, chimeric antibodies and humanized antibodies, which can be of different isotypes, e.g. IgG1.

The term "internalization" refers to the transport of a moiety from the outside to the inside of a cell. The internalized moiety can be located in an intracellular compartment, e.g. a vacuole, an endosome, a lysosome, the endoplasmic reticulum, the Golgi apparatus, or in the cytosol. An antibody that is "internalized" or "internalizing" refers to an antibody that is capable of being transported from the outside to the inside of a target cell.

The term "homogenous" when used herein in connection with a method or an assay means that the method or assay does not require physical separation or washing steps.

"High throughput" method as used herein refers to a method which provides for a large number of samples (e.g. 96 samples or more) to be screened simultaneously in a rapid and economical manner. Such methods may include the use of microtiter plates which are especially convenient because a large number of assays can be carried out simultaneously, using small amounts of reagents and samples. Often, such methods will be automated.

The term "fluorescent label" refers to any material capable of generating a detectable fluorescent signal.

The term "macro-confocal" scanning refers to optical interrogation with a focused beam of excitation light, wherein the optical interrogation is limited to a selected depth of field.

In a first main aspect, the invention relates to a method for selecting an antibody on the basis of its ability to be internalized into a cell, said method comprising the steps of

a) providing a plurality of antibodies to be tested for internalization, wherein each of said antibodies is provided as a separate sample, and wherein said antibodies bind the same antigen,
b) providing eukaryotic cells expressing the antigen to which said antibodies bind,
c) providing a detection tool capable of binding said antibodies, wherein said detection tool i) does not mediate internalization, and ii) comprises a fluorescent label,
d) incubating said samples, cells and detection tool in a test vial under conditions that allow formation and internalization of a complex comprising said antigen, antibody and detection tool,
e) detecting fluorescence of at least a cell-containing sub volume of said test vial, and
f) selecting an antibody on the basis of the outcome of the detection.

The method is suitable for high throughput screening. In one embodiment, all steps are carried out at a temperature between 18°C and 30°C, e.g. at room temperature.

Samples for the internalization method

Due to the use of a detection tool that recognizes any antibody, but does not mediate internalization itself, separate direct labeling of each antibody to be tested is avoided. Thus, the method of invention is much less laborious than methods known in the art, and thus, the assay can, in one embodiment, be used as a high-throughput assay in which many samples are analyzed simultaneously. Thus, in one embodiment, a plurality of samples, e.g. 96 or more samples, is analyzed simultaneously. For the same reason, the assay is suitable in early drug discovery, for direct hybridoma or transfectoma screening. Accordingly, in one embodiment, the samples provided are a plurality of samples derived from hybridoma cultures obtained from an immunization of an animal immunized with a particular antigen, wherein the purpose of the method of the invention is to identify, amongst these hybridoma cultures, ones that produce an antibody that is internalized or not internalized, depending on the contemplated use of the antibody. In another embodiment, the samples provided are from cultures of immortalized IgG-producing B cells, or IgG-producing transfectomas.

By choosing suitable concentrations of the cells and the dye, as exemplified in the Examples section herein, a highly sensitive assay is provided, allowing detection of internalization of even small amounts of antibody. Thus, the assay can be used to evaluate internalization directly in a supernatant of a hybridoma cell culture which only produces small amount of antibody. Thus, in one embodiment, the sample provided in step a) of the method is a sample that has not been concentrated to increase the antibody concentration, prior to being tested in the method of the invention.

In one embodiment, after addition of antibody and detection tool to the cells, the incubation mixture in step d) comprises 50 micrograms/ml of antibody or less, e.g. 5 micrograms/ml of antibody or less, such as 0.5 micrograms/ml antibody or less, e.g. 50 nanograms/ml or less, such as 5 nanograms/ml of antibody or less, e.g. 1 nanograms/ml of antibody or less, or even 0.1 nanograms/ml of antibody or less. In another embodiment, the incubation mixture comprises between 0.1 and 5, such as between 0.1 and 2 nanograms/ml of antibody.

Eukaryotic cells

The "eukaryotic cells expressing the antigen to which said antibodies bind" used in this invention include any type of eukaryotic cells capable of internalization of antibodies when placed under suitable conditions. The cells typically naturally express the antigen to which the antibody to be tested is directed, or they are transfected with a nucleic acid construct expressing the antigen. Thus, suitable cells also include cell lines transfected with a gene for a known target molecule, e.g. a receptor, to which it would be useful to have internalizing antibodies.

The cells can be cells from multicellular or unicellular eukaryotes. Preferred cells are mammalian cells, such as human cells. The cells can be normal healthy cells or cells characterized by a particular pathology (e.g. tumor cells).

Thus, in one embodiment, said cells are selected from the group consisting of: tumor cells or cell lines, such as A431, Daudi or Raji, lymphocytes and transfectoma cells.

Cells may be adherent or non-adherent, and the assay may be performed in any type of test vial, well, tube, suitable for fluorescence detection. Use of a multi-well format, e.g. a 96-well or 386-well format, allows simple identification of the antibody after detection.

Detection tool

The detection tool used in the internalization method of the invention is capable of binding the antibodies provided in step a) and comprises a fluorescent label for detection. Furthermore, the detection tool does not mediate internalization, i.e. it does not itself contain signals for internalization into the eukaryotic cells used in the assay.

In one embodiment, the detection tool used in the invention is a labeled Fab fragment capable of binding human antibodies. A Fab fragment is highly suitable as a detection tool, since it in itself does not mediate internalization, because it does not cross-link or contain an Fc part. Thus, in some embodiments, the detection tool is e.g. a Fab fragment of a goat anti human IgG or a rabbit anti human IgG. Other antibody fragments with similar characteristics may be equally suitable for use as detection tool.

In one embodiment, the detection tool used in the internalization method of the invention is labeled with a pH-sensitive dye, such as a red-excited fluorescent dye. In one embodiment, such a dye is a dye which is non-fluorescent at neutral pH and fluorescent at acidic pH. An example of such a dye is Cypher5 (Adie et al. (2002) Biotechniques 33, 1152). Another example is Cypher™5E (Beletskii et al. (2005) Biotechniques 39, 894). In one embodiment, the dye used is a dye which can be excited with a laser of 633 nm and which can display an emission within the range of 650-720 nm.

In a preferred embodiment, the detection tool used in the invention is a Fab fragment labeled with Cypher5 or Cypher™5E.

Detection of fluorescence

In its broadest aspect, the detection performed can be performed using any type of fluorescent detection method known in the art, e.g. using a PMT or CCD scanner. Examples of CCD scanners include the IN Cell Analyzer 1000 or 3000.

However, in preferred embodiments, detection is performed using a macro-confocal laser scanner which uses a laser scanner for excitation and a PMT scanner for detection. The use of a macro-confocal laser scanner allows the detection of only a subvolume of the test vial, here a subvolume containing the cells. This detection method ensures removal of background noise, allowing the assay to be run as a homogeneous assay, avoiding any washing steps. Also, no fixation and/or simultaneous nuclear staining is required.

Thus, while in some embodiments, the entire volume of the test vial is analyzed, in preferred embodiments, fluorescence is only detected from a cell-containing subvolume of said test vial.

In one embodiment, the detection in the method of the invention is performed using a macro-confocal laser scanner, such as the Applied Biosystems FMAT 8100 HTS or the Applied Biosystems 8200 Cellular Detection System.

For example, using the Applied Biosystems 8200 Cellular Detection System, the detection can be performed as follows: a 633 nm high speed laser scanner is used for excitation of fluorescent binding events. A 1 $mm^2$ area of a well (96,384 or 1536) is scanned with a depth of 100 micron at the plate bottom. Detection is performed using two PMT and a dichroic filter for two color events within the range of 650-720 nm. By using appropriate algorithms, background signal of free fluorescent label is subtracted to have discrete detection of binding events. Read out data from the system are fluorescence (FL1 and FL2) and counts. Fluorescence can be depicted as total or mean fluorescence per scanned area as well as fluorescence per event in the scanned area. An advantage of this type of detection system is that there is no auto fluorescence due to the use of the 633 nm with the particular PMT's and dichroic filter.

Selection of antibodies

In one embodiment, the method of the invention is performed in order to identify antibodies that are not internalized by the cells used in the assay. Thus, in such embodiments, an antibody that is not internalized is selected in step f). Antibodies that are not internalized may e.g. be preferred in therapeutic applications where effector mechanisms, such as CDC and/or ADCC are responsible for the therapeutic effect. Since internalization reduces the amount of antibody on the cell surface, it may cause reduced recognition of the target cell for activation of CDC and/or ADCC.

In other embodiment, the method of the invention is performed in order to identify antibodies that are internalized by the cells used in the assay. Thus, in such embodiments, an antibody that is internalized is selected in step f). Antibodies that are internalized may e.g. be preferred in therapeutic applications where receptor downregulation is desirable or conjugation of the antibody with e.g. a radio-isotope or other cytotoxic compounds is envisaged.

CDC and ADCC assays

Also disclosed herein is a method for selecting an antibody on the basis of its ability to mediate complement-dependent cytotoxicity, said method comprising the steps of

a) providing a plurality of antibodies to be tested for their capacity to mediate complement-dependent cytotoxicity, wherein each of said antibodies is provided as a separate sample, and wherein said antibodies bind the same antigen,
b) providing target cells expressing the antigen to which said antibodies bind,
c) providing complement,
d) incubating said sample, target cells and complement under conditions that allow complement activation and target cell lysis,
e) adding a first and a second dye, wherein the first dye is an anthraquinone or derivative thereof, and the second dye is a cyanine dye composed of a quinoline and a benzothiazole moiety, and
f) performing count measurements of individual cells, wherein the reading discriminates between cells that are stained with both the first and the second dye, and cells that are only stained with the first dye, wherein the measurement is performed using a macro-confocal laser scanner,

g) calculating the relative proportion of dead cells, and

h) selecting an antibody on the basis of the outcome of the calculation.

Also disclosed herein is a method for selecting an antibody on the basis of its ability to mediate antibody-dependent cellular cytotoxicity, said method comprising the steps of

a) providing a plurality of antibodies to be tested for their capacity to mediate antibody-dependent cellular cytotoxicity, wherein each of said antibodies is provided as a separate sample, and wherein said antibodies bind the same antigen,

b) providing target cells expressing the antigen to which said antibodies bind, wherein said target cells have been stained by pre-incubation with a first dye, wherein said first dye is an anthraquinone or derivative thereof,

c) providing effector cells,

d) incubating said sample, target cells and effector cells under conditions that allow effector cell activation and target cell lysis,

e) adding a second dye, wherein said second dye is a cyanine dye composed of a quinoline and a benzothiazole moiety,

f) performing count measurements of individual cells, wherein the reading discriminates between cells that are stained with both the first and the second dye, and cells that are only stained with the first dye, wherein the measurement is performed using a macro-confocal laser scanner,

g) calculating the relative proportion of dead cells, and

h) selecting an antibody on the basis of the outcome of the calculation.

Samples for the CDC and ADCC methods

The methods are much less laborious than methods known in the art, and thus, the assay can be used as a high-throughput assay in which many samples are analyzed simultaneously. Thus, a plurality of samples, e.g. 96 or more samples is analyzed simultaneously. For the same reason, the assay is suitable in early drug discovery, for direct hybridoma or transfectoma screening. Accordingly, the samples provided are a plurality of samples derived from hybridoma cultures obtained from an immunization of an animal immunized with a particular antigen and hybridoma fusion technology, wherein the purpose of the method is to identify amongst these hybridoma cultures, ones that produce an antibody that activates CDC or ADCC or an antibody that does not activate CDC and/or ADCC, depending on the contemplated use of the antibody. The samples provided may also be from cultures of immortalized IgG-producing B cells, or IgG-producing transfectomas.

By choosing suitable concentrations of the cells and the dye, as exemplified in the Examples section herein, a highly sensitive assay is obtained, allowing detection of complement activation of even small amounts of antibody. Thus, the assay can be used to evaluate complement activation directly in a supernatant of a hybridoma cell culture which only produces small amount of antibody. Thus, as disclosed herein, the sample provided in step a) of the method may be a sample that has not been concentrated to increase the antibody concentration.

Also disclosed herein, after addition of antibody and detection tool to the cells, the incubation mixture in step d) comprises 10 micrograms/ml of antibody or less, e.g. 5 micrograms/ml of antibody of less, such as 0.5 micrograms/ml antibody or less, e.g. 50 nanograms/ml or less, such as 5 nanograms/ml of antibody or less, e.g. 1 nanograms/ml of antibody or less, or even 0.1 nanograms/ml of antibody or less. Also disclosed herein, the incubation mixture comprises between 0.1 and 5, such as between 0.1 and 2 nanograms/ml of antibody.

Target cells

The "target cells expressing the antigen to which said antibodies bind" used in the methods of the invention include any type of cells capable of expressing the antigen of interest, and being susceptible to CDC and/or ADCC when placed under suitable conditions. The cells typically naturally express the antigen to which the antibody to be tested is directed, or they are transfected with a nucleic acid construct expressing the antigen. Thus, suitable cells also include cell lines transfected with a gene for a known target molecule, e.g. a receptor, to which it would be useful to have CDC-activating and/or ADCC-activating antibodies.

The cells can be cells from multicellular or unicellular eukaryotes. Preferred cells are mammalian cells, such as human cells. The cells can be normal healthy cells or cells characterized by a particular pathology (e.g. tumor cells).

Thus, in one embodiment, said cells are selected from the group consisting of: tumor cells or cell lines, such as A431, Daudi or Raji, lymphocytes and transfectoma cells.

Cells may be adherent or non-adherent, and the assay may be performed in any type of test vial, well, tube, suitable for fluorescence detection. Use of a multi-well format, e.g. a 96-well format, allows simple selection of the antibody after detection.

Effector cells for ADCC method

Effector cells in the ADCC method are cells that are capable of effecting ADCC. Disclosed herein, effector cells may be added in an effector-cell-to-target-cell ratio of 100:1 or less, such as 50:1 or less, e.g. 10:1 or less, such as 5:1 or less. Also disclosed herein, said ratio is between 200:1 and 1:1, such as between 100:1 and 10:1.

Different types of effector cells may be used. Examples of suitable cell type include purified NK cells, peripheral blood mononuclear cells (PBMC), purified PMN and monocyte/macrophages. In one embodiment, the incubation time with the effector cells is 2 hours or more, such as 4 hours or more, e.g. 10 hours or more, or 16 hours or more.

Dyes

An anthraquinone or derivative thereof is used in the CDC and ADCC methods of the invention as a (first) dye to stain all cells, i.e. live and dead cells. These dyes were found to be particularly useful in the method of the invention, due to amongst others, stable and homogenous staining of all cells. Examples of such dyes have been described in US 2006/0148777.. In one embodiment, the dye used is a compound as defined in sections 0004 to 0007 of US 2006/0148777 or a compound as defined in section 0010 of US 2006/0148777. In one embodiment, said first dye is DRAQ5™ or APOTRAK™.

A cyanine dye composed of a quinoline and a benzothiazole moiety is used in the CDC and ADCC methods as a (second) dye to stain dead cells. Such dyes have e.g. been described by Sovenyhazy et al. (2003) Nucl. Acids Res. 31:2561. In one embodiment, the second dye used is TOPRO-3.

Detection of fluorescence

Detection of fluorescence in the CDC and ADCC methods of the invention is done by performing count measurements of individual cells, wherein the reading discriminates between cells that are stained with both the first and the second dye, and cells that are only stained with the first dye. The measurement is performed using a macro-confocal laser scanner. As describe above, the use of a macro-confocal laser scanner allows the detection of only a subvolume of the test vial, here a subvolume containing the cells. This detection method ensures removal of background noise, allowing the assay to be run as a homogeneous assay, avoiding any washing steps.

Thus, fluorescence is only detected from a cell-containing subvolume of said test vial.

In one embodiment, the detection in the method of the invention is performed using a macro-confocal laser scanner such as the Applied Biosystems FMAT 8100 HTS or the Applied Biosystems 8200 Cellular Detection System. As described above, using the Applied Biosystems 8200 Cellular Detection System, the detection can be performed as follows: a 633 nm high speed laser scanner is used for excitation of fluorescent binding events. A 1 mm$^2$ area of a well (96,384 or 1536) is scanned with a depth of 100 micron at the plate bottom. Detection is performed using two PMT and a dichroic filter for two color events within the range of 650-720 nm. By using appropriate algorithms, background signal of free fluorescent label is subtracted to have discrete detection of binding events. Read out data from the system are fluorescence (FL1 and FL2) and counts. Fluorescence can be depicted as total or mean fluorescence per scanned area as well as fluorescence per event in the scanned area. An advantage of this type of detection system is that there is no auto fluorescence due to the use of the 633 nm with the particular PMT's and dichroic filter.

In one embodiment of the CDC and ACC methods of the invention, substantially all (e.g. more than 95% or more than 99% of the) individual cells that are present in the subvolume that is analyzed by the detector are being counted.

Selection of antibodies

Also disclosed herein, the CDC method may be performed in order to identify antibodies that activate CDC. Thus, an antibody that activates CDC is selected in step f). Antibodies that activates may e.g. be preferred in therapeutic applications where CDC is responsible for the therapeutic effect.

Also disclosed herein, the CDC method of the invention may be performed in order to identify antibodies that do not activate CDC. Thus, an antibody that does not activate is selected in step f). Antibodies that do not activate CDC may e.g. be preferred in therapeutic applications where only blocking of the target antigen and not killing of antigen-expressing cells is desired.

Also disclosed herein, the ADCC method of the invention is performed in order to identify antibodies that activate ADCC. Thus, an antibody that activates ADCC may be selected in step f). Antibodies that activates may e.g. be preferred in therapeutic applications where ADCC is responsible for the therapeutic effect.

Also disclosed herein, the ADCC method of the invention may be performed in order to identify antibodies that do not activate ADCC. Thus, an antibody that does not activate may be selected in step f). Antibodies that do not activate ADCC may e.g. be preferred in therapeutic applications where only blocking of the target antigen and not killing of antigen

expressing cells is desired.

Further aspects

Also disclosed herein is an antibody selected by any of the methods of the invention described herein. Also disclosed herein is an antibody selected by any of the methods of the invention for use as a medicament. In a further aspect, one or more of the methods of the invention are combined in order to select, from a plurality of antibodies, one or more antibodies that are suitable for use as a medicament.

Accordingly, in a further aspect, the invention relates to a method for selecting an antibody suitable for the treatment of disease, comprising performing, on a plurality of samples, two or all three of the methods selected from the group consisting of:

- the internalization methods as described herein,
- the CDC methods as described herein, and
- the ADCC methods as described herein.

For example, in one embodiment, a plurality of antibody samples may be tested for internalization as well as for CDC activation, in order to identify an antibody that is not internalized and does activate CDC. In another embodiment, a plurality of antibodies samples may be tested for internalization as well as for ADCC activation, in order to identify an antibody that is not internalized and does activate ADCC.

Examples of antibodies to be tested in the internalization-, CDC- and/or ADCC-method of the present invention include antibodies that bind antigens such as leukocyte surface markers or CD proteins e.g. CD1 a-c, CD2, CD2R, CD3, CD4, CD5, CD6, CD7, CD8, CD9, CD10, CD11a, CD11b, CD11c, CDw12, CD13, CD14, CD15, CD15s, CD16, CD16b, CDw17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, C41, CD42a-d, CD43, CD44, CD44R, CD45, CD45A, CD45B, CD450, CD46-CD48, CD49a-f, CD50, CD51, CD52, CD53-CD59, CDw60, CD61, CD62E, CD62L, CD62P, CD63, CD64, CDw65, CD66a-e, CD68-CD74, CDw75, CDw76, CD77, CDw78, CD79a-b, CD80-CD83, CDw84, CD85-CD89, CDw90, CD91, CDw92, CD93-CD98, CD99, CD99R, CD100, CDw101, CD102-CD106, CD107a-b, CDw108, CDw109, CD115, CDw116, CD117, CD119, CD120a-b, CD121a-b, CD122, CDw124, CD126-CD129, and CD130; members of the ErbB receptor family such as the EGF receptor, HER2 receptor, ErbB3 receptor or ErbB4 receptor; prostate specific antigen(s); and cell adhesion molecules such as IIb/IIIa, LFA-1, Mac1, p150.95, VLA-4, ICAM-1, and VCAM.

**EXAMPLES**

The present invention is further illustrated by the following examples which should not be construed as further limiting.

**EXAMPLE 1: INTERNALIZATION ASSAYS**

**Antibodies**

| Antibody | Parental/clone | Batch code | Source |
|---|---|---|---|
| HuMabCD38-049 | P3003-049-1 C7 | 0472-001-EP | Genmab |
| HuMab-KLH | LC0180-001 | 0391-020-EP | Genmab |
| Fab-fragment goat anti-human IgG (H+L specific) | 109-007-003 | 65085 | Jackson |

HuMabCD38-049 is a human monoclonal IgG1,κ antibody capable of binding human CD38. The antibody was generated as follows:

Human immunoglobulin transgenic HCo12 mice were immunized once every fourteen days with 20 μg purified HA-CD38 alternating with NIH-3T3-CD38 transfected cells. The first immunization was performed with 5 x $10^6$ cells in 100 μl PBS, mixed with 100 μl CFA, i.p., the second and following immunizations with HA-CD38 s.c., in the presence of 100 μl PBS, mixed with 100 μl IFA. The following immunizations with transfected cells were performed in the presence of 200 μl PBS. After titer development, mice were boosted with 20 μg HA-CD38 in PBS, i.v.

Mouse splenocytes were isolated from the immunized HCo12 mice and fused with PEG to a mouse myeloma cell line based upon standard protocols. The resulting hybridomas were then screened for human antibody production by ELISA and for CD38 specificity using CHO-CD38 cells by FACS analysis and recombinant HA-CD38 protein binding by ELISA. Hybridoma cell line -049, expressing human monoclonal anti-CD38 antibodies, was selected for these studies. The produced antibody, HuMabCD38-049 cross-competes for CD38 binding with antibody -005, described in WO 2006/099875 (Genmab), but not with antibody -003, also described in WO2006/099875. Antibodies were purified as described in the Examples section of WO2006/099875.

HuMab-KLH is a human monoclonal IgG1,κ antibody against KLH (keyhole limpet haemocyanin).

**Reagents, Chemicals and Consumables**

| Product | Cat. Nr. | Supplier | Lot. No. or Experiment number |
|---|---|---|---|
| CHO-CD38 cells | na | Genmab | EX3003-0407-003 p60 |
| Daudi cells | na | Genmab | EX3003-0407-006 p21 |
| CypHer 5 mono NHS Ester | PA15401 | Amersham Biosciences | 301594 |
| 96 wells FMAT | 4308776 | Greiner | 00704013 |
| Sodium azide | 13412 | Sigma-Aldrich chemicals | 22460 |
| BSA fraction V | 735086 | Roche | 70027920 |

CHO-CD38 cells, Chinese Hamster Ovary cells expressing human CD38, were kindly provided by Prof. M. Glennie (Tenovus Research Laboratory, Southampton General Hospital, Southampton, UK. CHO-CD38 cells were cultured as described in the Examples section of WO2006/099875

Daudi-luc cells were generated as described in the Examples section of WO2006/099875. They were cultured in RPMI 1640 (Cambrex Bioscience, Verviers, Belgium) culture medium supplemented with 10% FCS (Optimum C241, Wisent Inc., St. Bruno, QC, Canada), 2 mM L-glutamine, 100 IU/ml penicillin, 100 mg/ml streptomycin, 1 mM sodium pyruvate (all purchased from Gibco BRL, Life Technologies, Paisley, Scotland). Medium was refreshed twice a week. Before use, cells were split and seeded at $1\text{-}1.5 \times 10^6$ cells/ml to ensure viability and optimal growth.

**Solutions**

10x PBS pH 7.4

1440 mg/l $KH_2PO_4$
90,000 mg/l NaCl
7950 mg/l $Na_2HPO_4$

FMAT buffer (with or without sodium azide)

200 ml 10X PBS filled up to almost 2 liter distilled water
Add 2 g BSA fraction V
Add 4 ml 10% Sodium Azide
Filled up to 2 liter with distilled water
Sterile filtered, degassed

10% BSA

10 g BSA
up to 100 ml with distilled water

10% Sodium azide

10 g Sodium Azide
up to 100 ml with distilled water

## Methods

Fab fragment GtaHuIgG (goat-anti-human IgG) was labeled with CypHer 5 according to instruction manual of Amersham Biosciences (Technical note: PA 15401/05PS Rev. B 2003). Before labeling, the protein concentration was determined using a Nanodrop® spectrophotometer. After labeling, the labeled Fab fragment was purified using dialysis and analyzed using an Ultrospec 2100™ pro spectrophotometer.

Cells were cultured as described above and plated at 5000 cells/well (100 $\mu$l/well) for overnight incubation at 37°C+ 5%$CO_2$. After overnight, incubation the medium was removed and a concentration range of antibody was added (15 $\mu$l/well). Subsequently, 560 ng/ml Fab GtaHuIgG-cypHer5 (25 $\mu$l/well) was added, and plates were incubated at 37°C for 2 hours before measurement. As a negative control for internalization, identical samples were kept on ice. The reaction was stopped using FMAT buffer+sodium azide.

Detection was performed using an Applied Biosystems 8200 Cellular Detection System.

In another set-up, 120 ng/ml Fab GtaHuIgG-cypHer5 (125 $\mu$l/well) was added, and plates were incubated for 9 hours at room temperature/24°C instead of overnight at 37°C. Otherwise, the assay conditions were the same.

## Results

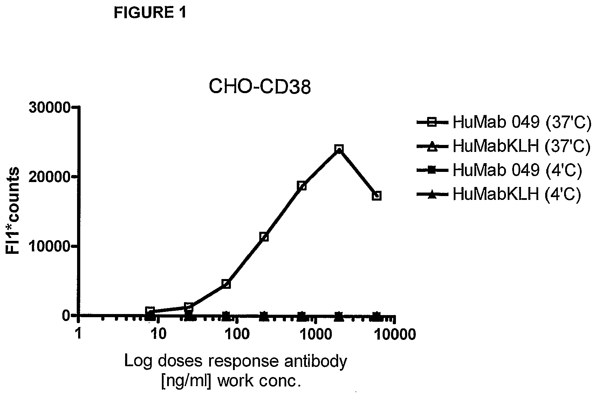

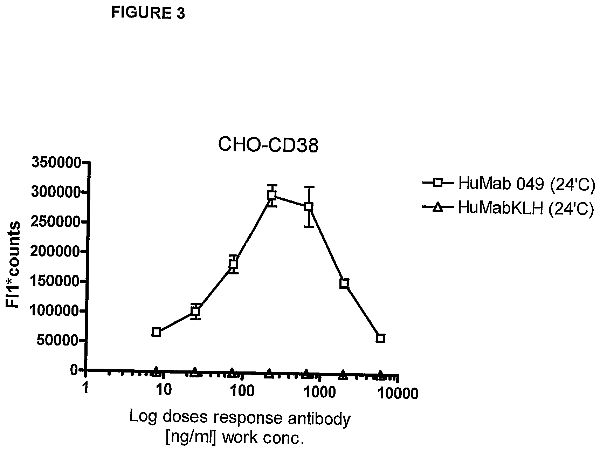

Fluorescence of the dye, indicative of cellular uptake of the antibody by internalization was found using CHO-CD38 cells both using overnight cultured cells (37°C) (figure 1) as well as fresh plated cells (24°C) (figure 3). No internalization was found in the negative control experiments (HuMab-KLH and 4°C).

Internalization was also detected using Daudi cells, although the counts were lower, possibly due to the cells being non-adherent.

The order of addition of cells, Fab GtaHuIgG-cypHer5 and antibody was tested, but found not to make any significant difference.

## EXAMPLE 2: CDC AND ADCC ASSAY

**Antibodies**

| Antibody | Parental/clone | Source |
|---|---|---|
| HuMab-KLH | 4A4 | Genmab |
| rituximab | | Roche |
| HuMab CD38 | S3003-005-012 | Genmab (see WO2006/099875) |
| HuMab CD38 | S3003-003-042 | Genmab (see WO2006/099875) |
| HuMab-CD38 | LC3003-048 | Genmab |

**Reagents, Chemicals and Consumables**

| Product | Cat. Nr. | Supplier | Lot. Nr |
|---|---|---|---|
| CHO-CD38 | na | Genmab | |
| Daudi | na | Genmab | |
| Daudi-luciferase | | Genmab | |
| 96 wells FMAT | 4308776 | Greiner | 00704013 |
| 10XPBS | 17-517Q | Cambrex | 4MB0102 |
| FMAT buffer | na | Genmab | 0552-047-MBH |
| Sodium azide | 13412 | Sigma-Aldrich chemicals | 22460 |
| BSA fraction V | 735086 | Roche | 70027920 |
| Chromepure | | Jackson Immuno | 67499 |

(continued)

| Product | Cat. Nr. | Supplier | Lot. Nr |
|---------|----------|----------|---------|
| DRAQ5 | DRAQ5 | Biostatus | BS2/2 |
| TOPRO-3-Iodide | T3605 | Molecular Probes | 4981-10 |

**Solutions**

10x PBS, FMAT buffer and 10% Sodium azide as described above.
Daudi-Luciferase medium: RPMI 1640, 10% Cosmic Calf Serum, 2 mM L-glutamine, 100 IU/ml penicillin, 100 mg/ml streptomycin, 1 mM sodium pyruvate
Daudi medium: RPMI 1640, 10% Cosmic Calf Serum, 2 mM L-glutamine, 100 IU/ml penicillin, 100 mg/ml streptomycin, 1 mM sodium pyruvate
CHO-CD38 medium: RPMI 1640, Cosmic Calf Serum, Sodium Pyruvate, Penicillin/Streptomycin, Puromycin.

**CDC method**

CHO-CD38, Daudi or Daudi-luciferase cells were plated into FMAT plates at a concentration of 25,000 cells per well (in 50 microliters of RPMI 1640 plus 10% BSA). Subsequently, 50 microliter antibody dilution in RPMI 1640 plus 10% BSA was added to each well, and the samples were incubated for 15 min at room temperature. This was followed by addition of 11 microliter Normal Human Serum to each well, and an incubation for 45 minutes at 37$\underline{o}$C, 5% CO2 and 80% humidity.
For staining, 25 microliters of DRAQ5 (0.5 microM), of TOPRO-3 (10 nanoM) or of TOPRO-3 plus DRAQ5 (10 nanoM / 0.5 microM) was added to the wells. The first and second dye can be added in any order. After 4-10 hours incubation, detection was performed using an Applied Biosystems 8200 Cellular Detection System.

**CDC data analysis method**

1) Gates (color plot) were set on DRAQ5 staining alone, and TOPRO-3 alone
2) From the double stain, two populations (A and B) were analyzed on counts:

    A. TOPRO-3 alone plus TOPRO-3/DRAQ5 stain
    B. DRAQ5 alone

3) % lysis was calculated using the following formula:

$$(\text{Counts Pop A}/(\text{Counts Pop A} + \text{Counts Pop B})) * 100$$

**CDC results**

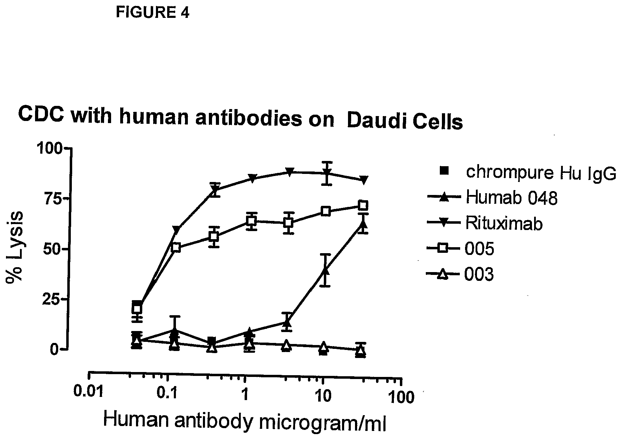

Figure 4 shows dose-response curves of CDC activity induced by different human antibodies on Daudi cells. The activity was measured on an Applied Biosystems 8200 Cellular Detection System using a dual stain of DRAQ5 and TOPRO-3. The graphs show clear differences in activity between the different antibodies. These differences are comparable with results from CDC analysis on FACS (not shown).

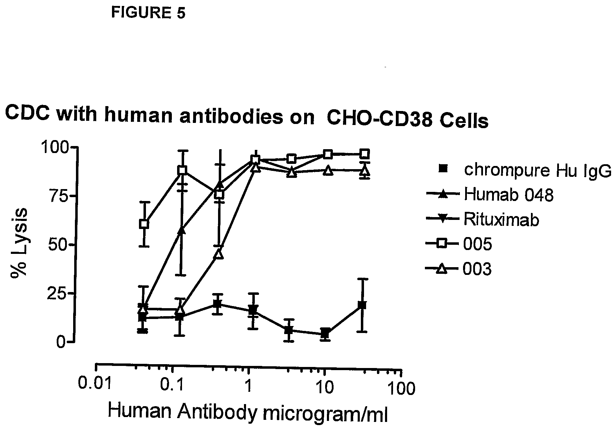

Figure 5 shows dose-response curves of CDC activity induced by different human antibodies on CHO-CD38 cells. The activity was measured on an Applied Biosystems 8200 Cellular Detection System using a dual stain of DRAQ5 and TOPRO-3. The graphs show clear differences in activity between the different antibodies. These differences are comparable with results from CDC analysis on FACS.

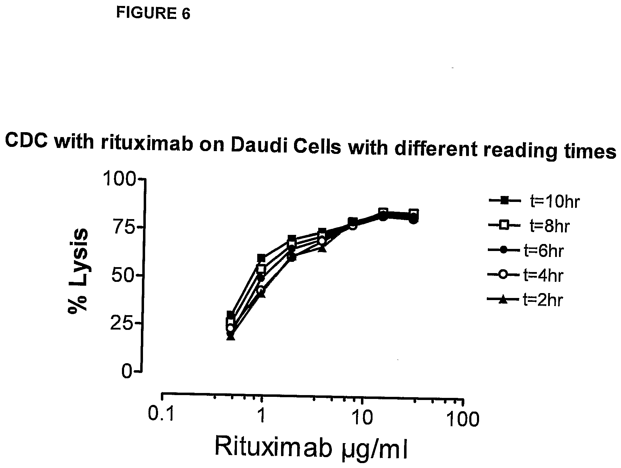

Figure 6 shows dose-response curves of CDC activity induced by rituximab on Daudi cells. After completion of the CDC by stopping the reaction with the Azide buffer the plate was measured at different time points. The activity was measured on an Applied Biosystems 8200 Cellular Detection System using a dual stain of DRAQ5 and TOPRO-3. Curves are similar from 2 to 10 hours and the maximal lysis stays more or less the same. Thus, time between assay and measure response does not influence the outcome dramatically.

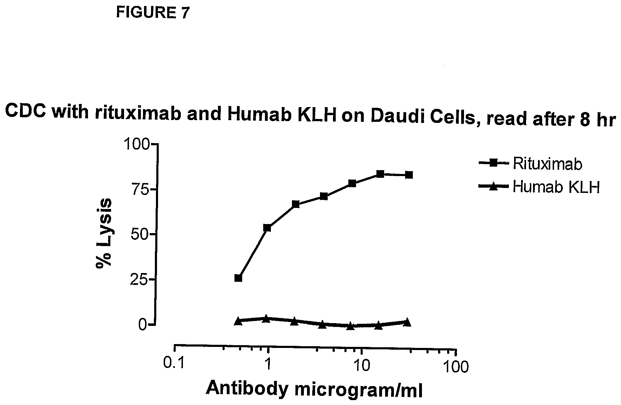

Figure 7 shows dose-response curves of CDC activity induced by rituximab and HuMab-KLH (negative control) on Daudi cells. The activity was measured on an Applied Biosystems 8200 Cellular Detection System using a dual stain of DRAQ5 and TOPRO-3. The graph shows that irrelevant antibodies do not induce cell lysis in the presence of complement. Over a time period of 8 hours the azide buffer does not induce TOPRO-3 stain due to aspecific (not antibody) related cell death. Thus, the complement cell lysis with rituximab can be interpreted as rituximab specific lysis.

It can be concluded that a double stain with DRAQ5 and TOPRO-3 and detection using macro-confocal laser scanning can be used to measure CDC activity of monoclonal antibodies. The data obtained with this assay correspond with respect to activity (EC50) and maximal lysis to those obtained using FACS (not shown).

**ADCC method**

Daudi-luc cells are collected ($2x10^5$ cells/ml) in RPMI$^{++}$ (RPMI 1640 culture medium supplemented with 10% cosmic calf serum (HyClone, Logan, UT, USA)). Subsequently, DRAQ5 is added to 90 nM, and the mixture is incubated in at 37°C, 5% $CO_2$ and 80% for 1 hr. After washing of the cells (thrice in RPMI$^{++}$, 1500 rpm, 5 min), the cells are resuspended in RPMI$^{++}$ at a concentration of approx $1x10^5$ cells/ml.

Preparation of effector cells

Fresh peripheral blood mononuclear cells (healthy volunteers, UMC Utrecht, Utrecht, The Netherlands) are isolated from 40 ml of heparin blood by Ficoll (Bio Whittaker; lymphocyte separation medium, cat 17-829E) according to the manufacturer's instructions. After resuspension of cells in RPMI$^{++}$, cells are counted by trypan blue exclusion and brought to a concentration of $1x10^7$ cells/ml.

ADCC set up

50 $\mu$l of DRAQ5-labeled target cells are pipetted into 96-well plates, and 50 $\mu$l of antibody is added, diluted in RPMI$^{++}$ (final concentrations 10, 1, 0.1, 0.01 $\mu$g/ml). Cells are incubated (RT, 15 min), and 50 $\mu$l effector cells are added, resulting in an effector-cell-to-target-cell-ratio of 100:1. Cells are incubated for 4 hours at 37°C, 5% $CO_2$. After incubation, 25 microliter TOPRO-3 solution (stock solution of about 10-2.5 nanoM) is added.

The assay is read on the Applied Biosystems 8200 Cellular Detection system, and data analysis is performed by gating on color plots for the different dyes. The percentage of specific lysis is calculated as follows:

$$\text{(Cell count DRAQ5 plus TOPRO-3 positive cells)/ (Cell count DRAQ5 plus TOPRO-3 positive cells + Cell count DRAQ5 positive cells) *100}$$

**EXAMPLE 3: ANTIBODY GROUP CLASSIFICATION VIA INTERNALIZATION**

**Antibodies used:**

| Antibody | Parental/Clone | Cross block group |
|---|---|---|
| Humab CD38 | S3003-005-012 (see also WO2006/099875) | 3 |
| Humab CD38 | S3003-003-042 (see also WO2006/099875) | 2 |
| Humab CD38 | LC3003-012 | 2 |
| Humab CD38 | LC3003-018 | 1 |
| Humab CD38 | P3003-025-1E11 | 3 |
| Humab CD38 | P3003-026 | 3 |
| Humab CD38 | LC3003-028 | 3 |
| Humab CD38 | LC3003-029 | 4 |
| Humab CD38 | LC3003-031 | 4 |
| Humab CD38 | P3003-034-2G11 | 3 |

(continued)

| Antibody | Parental/Clone | Cross block group |
|---|---|---|
| Humab CD38 | LC3003-048 | 4 |
| Humab CD38 | P3003-049-1 C7 | 3 |
| Humab CD38 | LC3003-072 | 2 |
| Humab CD38 | LC3003-075 | 3 |

**Methods:**

Fab fragments were labeled as described in Example 1. CHO-CD38 cells were cultured as described in Example 1. Cells were collected and resuspended in FMAT buffer without sodium azide . Fab anti HuIgG-Cypher5 was added to the cell suspension in a final concentration of 120 ng/ml. Assay plates were filled with 125 $\mu$l/well of the cell suspension. Subsequently 15 $\mu$l/well of sample was added. After 6 hours of incubation at roomtemperature/24°C, detection was performed on the Applied Biosystems 8200 Cellular Detection System. Cross-blocking of antibodies was determined using FACS as described in Example 7 of WO2006/099875.

**Results:**

Human anti human-CD38 antibodies from different cross block groups were tested for internalization capabilities with the above described indirect internalization assay using the Applied Biosystems 8200 Cellular Detection System. The samples were tested individually in dose response in duplicates.

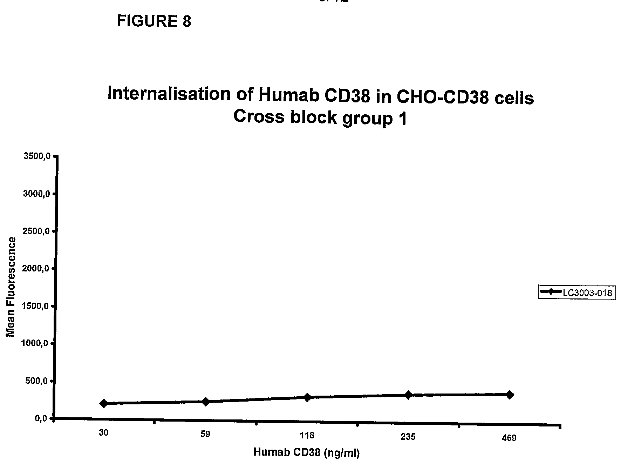

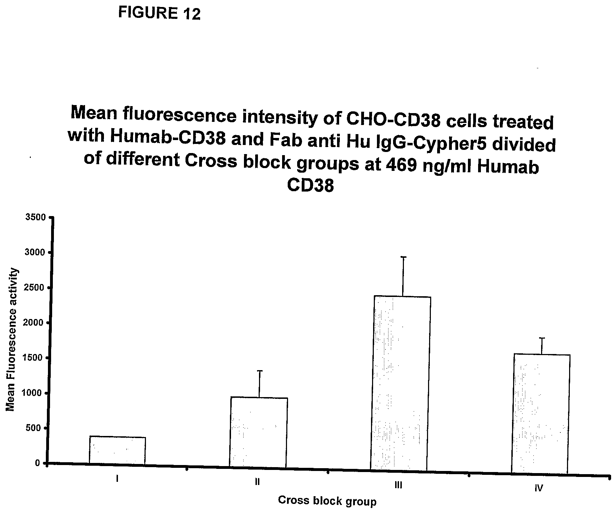

Figures 8 to 11 show different internalization capabilities for the tested antibodies. Each figure contains data of one cross-block group. Internalization is depicted as mean fluorescence per well +/- SEM. In figure 12 the mean fluorescence intensity for each group is depicted with SEM.

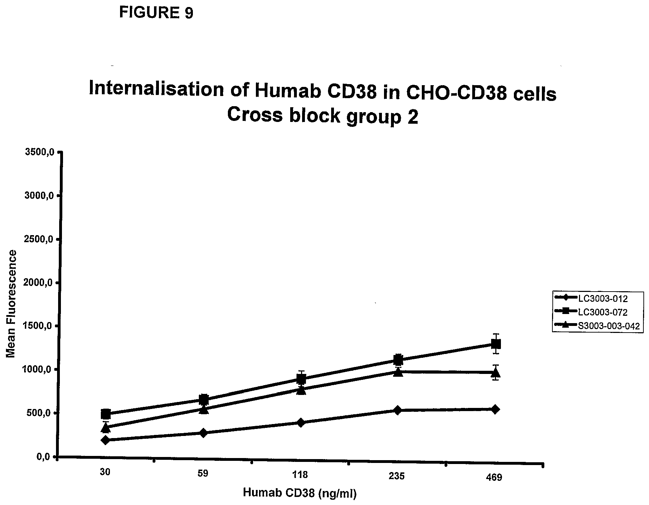

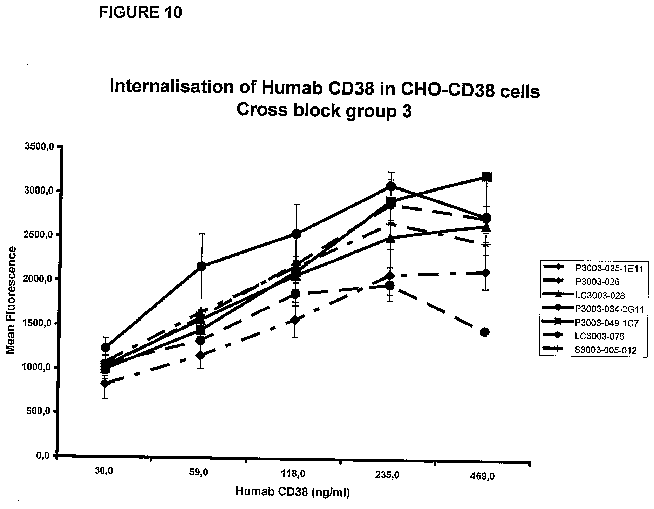

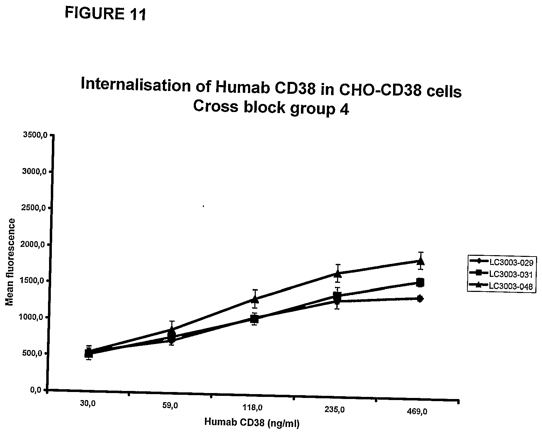

There are clearly distinct internalization capabilities between antibodies and cross block groups. The figures show a rank order for the different cross block groups. Group 3 (fig. 10) shows the highest internalization capabilities followed by group 4 (fig. 11). The antibodies of group 2 (Fig. 9) are less capable of internalization than those of groups 3 and 4, but better than those of group 1 (Fig. 8). Figure 12 shows clearly that the internalization capabilities differ per cross-block group.

**Claims**

1.  A method for selecting an antibody on the basis of its ability to be internalized into a cell, said method comprising the steps of

    a) providing a plurality of antibodies to be tested for internalization, wherein each of said antibodies is provided as a separate sample, and wherein said antibodies bind the same antigen,
    b) providing eukaryotic cells expressing the antigen to which said antibodies bind,
    c) providing a Fab fragment capable of binding said antibodies, wherein said Fab fragment i) does not mediate internalization, and ii) comprises a fluorescent label,
    d) incubating said samples, cells and Fab fragment in a test vial under conditions that allow formation and internalization of a complex comprising said antigen, antibody and Fab fragment,
    e) detecting fluorescence of at least a cell-containing subvolume of said test vial, and
    f) selecting an antibody on the basis of the outcome of the detection.

2.  The method of claim 1, wherein detection of a cell-containing subvolume of said test vial is performed using a macro-confocal laser scanner.

3.  The method of any of the preceding claims, wherein said method is a homogeneous method.

4.  The method of any of the preceding claims, wherein said sample is a supernatant of a cell culture of hybridomas, of immortalized IgG-producing B cells, or of IgG-producing transfectomas.

5.  The method of any of the preceding claims, wherein said sample has not been concentrated prior to being used in the method.

6. The method of any of the preceding claims, wherein the incubation mixture in step d) comprises 50 micrograms/ml of antibody or less, e.g. 5 micrograms/ml of antibody or less, such as 0.5 micrograms/ml antibody or less, e.g. 50 nanograms/ml or less, such as 5 nanograms/ml of antibody or less.

7. The method of any of the preceding claims, wherein the incubation mixture in step d) comprises 1 nanogram/ml of antibody or less.

8. The method of any of the preceding claims, wherein the incubation mixture in step d) comprises 0.1 nanogram/ml of antibody or less.

9. The method of any of the preceding claims, wherein said eukaryotic cells are selected from the group consisting of: tumor cells or cell lines, such as A431, Daudi or Raji, lymphocytes and transfectoma cells.

10. The method of any of the preceding claims, wherein said fluorescent label is a pH-sensitive dye.

11. The method of any of the preceding claims, wherein said label is Cypher5 or CypHer5E, and wherein the selection in step f) comprises selecting an antibody that gives rise to fluorescence if internalization is desired, and selecting an antibody that does not give rise to fluorescence, if no internalization is desired.

12. The method of any of the preceding claims, wherein said detection in step e) comprises the use of software to subtract background fluorescence.

13. The method of any of the preceding claims, wherein said detection in step e) is performed using an Applied Biosystems 8200 Cellular Detection System.

14. The method of any of the preceding claims, wherein an antibody that is not internalized is selected in step f).

15. The method of any of claims 1 to 13, wherein an antibody that is internalized is selected in step f).

16. A method for selecting an antibody suitable for the treatment of disease, comprising performing, on a plurality of samples:

   - the method of any of claims 1 to 15.

**Patentansprüche**

1. Verfahren zur Auswahl eines Antikörpers auf Basis seiner Fähigkeit, in eine Zelle internalisiert zu werden, wobei das Verfahren folgende Schritte umfasst

   a) Bereitstellen einer Vielzahl von Antikörpern, um sie auf ihre Internalisierung hin zu testen, wobei jeder dieser Antikörper als separate Probe bereitgestellt wird und wobei die Antikörper dasselbe Antigen binden,
   b) Bereitstellen eukaryotischer Zellen, welche das Antigen exprimieren, an welches die Antikörper binden,
   c) Bereitstellen eines Fab-Fragments, welches in der Lage ist, die Antikörper zu binden, wobei das Fab-Fragment i) keine Internalisierung vermittelt und ii) eine fluoreszierende Markierung umfasst,
   e) Inkubieren der Probenzellen und des Fab-Fragments in einem Testgefäß unter Bedingungen, welche die Bildung und Internalisierung eines Komplexes, umfassend das Antigen, den Antikörper und das Fab-Fragment, erlauben,
   d) Detektieren von Fluoreszenz wenigstens eines Zell-enthaltenden Subvolumens des Testgefäßes und
   f) Auswählen eines Antikörpers auf Basis des Ergebnisses der Detektion.

2. Verfahren gemäß Anspruch 1, wobei die Detektion eines Zell-enthaltenden Subvolumens des Testgefäßes unter Verwendung eines Makro-Konfokal-Laserscanners ausgeführt wird.

3. Verfahren gemäß einem der vorherigen Ansprüche, wobei das Verfahren ein homogenes Verfahren ist.

**4.** Verfahren gemäß einem der vorherigen Ansprüche, wobei die Probe ein Überstand einer Zellkultur von Hybridomen, immortalisierten IgG-produzierenden B-Zellen oder von IgG-produzierenden Transfektomen ist.

**5.** Verfahren gemäß einem der vorherigen Ansprüche, wobei die Probe vor ihrer Verwendung im Verfahren nicht konzentriert worden ist.

**6.** Verfahren gemäß einem der vorherigen Ansprüche, wobei die Inkubationsmischung in Schritt d) 50 Mikrogramm/ml Antikörper oder weniger umfasst, zum Beispiel 5 Mikrogramm/ml Antikörper oder weniger, wie etwa 0,5 Mikrogramm/ml Antikörper oder weniger, zum Beispiel 50 Nanogramm/ml oder weniger, wie etwa 5 Nanogramm/ml Antikörper oder weniger.

**7.** Verfahren gemäß einem der vorherigen Ansprüche, wobei die Inkubationsmischung in Schritt d) 1 Nanogramm/ml Antikörper oder weniger umfasst.

**8.** Verfahren gemäß einem der vorherigen Ansprüche, wobei die Inkubationsmischung in Schritt d) 0,1 Nanogramm/ml Antikörper oder weniger umfasst.

**9.** Verfahren gemäß einem der vorherigen Ansprüche, wobei die eukaryotischen Zellen ausgewählt werden aus der Gruppe, bestehend aus: Tumorzellen oder -zelllinien, wie etwa A431, Daudi oder Raji, Lymphozyten und Transfektomzellen.

**10.** Verfahren gemäß einem der vorherigen Ansprüche, wobei die fluoreszierende Markierung ein pH-empfindlicher Farbstoff ist.

**11.** Verfahren gemäß einem der vorherigen Ansprüche, wobei die Markierung Cypher5 oder CypHer5E ist und wobei die Auswahl in Schritt f) umfasst: Auswählen eines Antikörpers, welcher zu Fluoreszenz führt, falls die Internalisierung gewünscht ist, und Auswählen eines Antikörpers, welcher nicht zu Fluoreszenz führt, falls keine Internalisierung gewünscht ist.

**12.** Verfahren gemäß einem der vorherigen Ansprüche, wobei die Detektion in Schritt e) umfasst die Verwendung von Software, um Hintergrundfluoreszenz abzuziehen.

**13.** Verfahren gemäß einem der vorherigen Ansprüche, wobei die Detektion in Schritt e) unter Verwendung eines Applied Biosystems 8200 Cellular Detection System ausgeführt wird.

**14.** Verfahren gemäß einem der vorherigen Ansprüche, wobei ein Antikörper, welcher nicht internalisiert ist, in Schritt f) ausgewählt wird.

**15.** Verfahren gemäß einem der Ansprüche 1 bis 13, wobei ein Antikörper, welcher internalisiert wird, in Schritt f) ausgewählt wird.

**16.** Verfahren zur Auswahl eines Antikörpers, welcher geeignet ist zur Behandlung einer Krankheit, umfassend die Durchführung an einer Vielzahl von Proben des Verfahrens nach einem der Ansprüche 1 bis 15.

**Revendications**

**1.** Procédé de sélection d'un anticorps sur la base de son aptitude à être internalisé dans une cellule, ledit procédé comprenant les étapes consistant à

a) fournir une pluralité d'anticorps à tester pour l'internalisation, chacun desdits anticorps étant fourni sous forme d'un échantillon distinct, et lesdits anticorps se fixant au même antigène,
b) fournir des cellules eucaryotes exprimant l'antigène auquel se fixent lesdits anticorps,
c) fournir un fragment Fab capable de se lier auxdits anticorps, lequel fragment Fab i) n'induit pas d'internalisation et ii) comprend un marqueur fluorescent,
d) mettre à incuber dans un flacon à essai lesdits échantillons, lesdites cellules et ledit fragment Fab dans des conditions qui permettent la formation et l'internalisation d'un complexe comprenant lesdits antigène, anticorps et fragment Fab,

e) détecter la fluorescence d'au moins un volume partiel contenant des cellules dudit flacon à essai, et

f) sélectionner un anticorps sur la base du résultat de la détection.

2. Procédé selon la revendication 1, dans lequel on effectue la détection d'un volume partiel contenant des cellules dudit flacon à essai en utilisant un scanner laser macro-confocal.

3. Procédé selon l'une quelconque des revendications précédentes, ledit procédé étant un procédé homogène.

4. Procédé selon l'une quelconque des revendications précédentes, dans lequel ledit échantillon est un surnageant d'une culture cellulaire d'hybridomes, de lymphocytes B immortalisés producteurs d'IgG ou de transfectomes producteurs d'IgG.

5. Procédé selon l'une quelconque des revendications précédentes, dans lequel ledit échantillon n'a pas été concentré avant d'être utilisé dans le procédé.

6. Procédé selon l'une quelconque des revendications précédentes, dans lequel le mélange d'incubation dans l'étape d) comprend 50 microgrammes/ml d'anticorps ou moins, par exemple 5 microgrammes/ml d'anticorps ou moins, comme 0,5 microgramme/ml d'anticorps ou moins, par exemple 50 nanogrammes/ml ou moins, comme 5 nanogrammes/ml d'anticorps ou moins.

7. Procédé selon l'une quelconque des revendications précédentes, dans lequel le mélange d'incubation dans l'étape d) comprend 1 nanogramme/ml d'anticorps ou moins.

8. Procédé selon l'une quelconque des revendications précédentes, dans lequel le mélange d'incubation dans l'étape d) comprend 0,1 nanogramme/ml d'anticorps ou moins.

9. Procédé selon l'une quelconque des revendications précédentes, dans lequel lesdites cellules eucaryotes sont choisies dans l'ensemble constitué par des cellules tumorales ou des lignées cellulaires, telles que A431, Daudi ou Raji, des lymphocytes et des transfectomes.

10. Procédé selon l'une quelconque des revendications précédentes, dans lequel ledit marqueur fluorescent est un colorant sensible au pH.

11. Procédé selon l'une quelconque des revendications précédentes, dans lequel ledit marqueur est Cypher5 ou CypHer5E, et dans lequel la sélection dans l'étape f) comprend la sélection d'un anticorps qui provoque une fluorescence si une internalisation est souhaitée, et la sélection d'un anticorps qui ne provoque pas de fluorescence si aucune internalisation n'est souhaitée.

12. Procédé selon l'une quelconque des revendications précédentes, dans lequel ladite détection dans l'étape e) comprend l'utilisation d'un programme pour soustraire la fluorescence de fond.

13. Procédé selon l'une quelconque des revendications précédentes, dans lequel on effectue ladite détection dans l'étape e) en utilisant un système de détection Biosystems 8200 Cellular Detection System

14. Procédé selon l'une quelconque des revendications précédentes, dans lequel on sélectionne dans l'étape f) un anticorps qui n'est pas internalisé.

15. Procédé selon l'une quelconque des revendications 1 à 13, dans lequel on sélectionne dans l'étape f) un anticorps qui est internalisé.

16. Procédé pour la sélection d'un anticorps approprié au traitement d'une maladie, comprenant l'exécution, sur une pluralité d'échantillons,

- du procédé selon l'une quelconque des revendications 1 à 15.

**FIGURE 1**

CHO-CD38

Legend:
- HuMab 049 (37'C)
- HuMabKLH (37'C)
- HuMab 049 (4'C)
- HuMabKLH (4'C)

X-axis: Log doses response antibody [ng/ml] work conc.

Y-axis: FI1*counts

FIGURE 2

DAUDI

**FIGURE 3**

CHO-CD38

HuMab 049 (24'C)
HuMabKLH (24'C)

Log doses response antibody
[ng/ml] work conc.

FIGURE 4

## CDC with human antibodies on Daudi Cells

FIGURE 5

CDC with human antibodies on CHO-CD38 Cells

Legend:
- ■ chrompure Hu IgG
- ▲ Humab 048
- ▼ Rituximab
- □ 005
- △ 003

X-axis: Human Antibody microgram/ml
Y-axis: % Lysis

**FIGURE 6**

CDC with rituximab on Daudi Cells with different reading times

FIGURE 7

## CDC with rituximab and Humab KLH on Daudi Cells, read after 8 hr

FIGURE 8

Internalisation of Humab CD38 in CHO-CD38 cells
Cross block group 1

FIGURE 9

**Internalisation of Humab CD38 in CHO-CD38 cells
Cross block group 2**

**FIGURE 10**

Internalisation of Humab CD38 in CHO-CD38 cells
Cross block group 3

FIGURE 11

## Internalisation of Humab CD38 in CHO-CD38 cells
## Cross block group 4

FIGURE 12

Mean fluorescence intensity of CHO-CD38 cells treated with Humab-CD38 and Fab anti Hu IgG-Cypher5 divided of different Cross block groups at 469 ng/ml Humab CD38

## REFERENCES CITED IN THE DESCRIPTION

### Patent documents cited in the description

- US 6794128 B
- US 7045283 B
- US 2004018198 A
- US 20060148777 A
- WO 2006099875 A

### Non-patent literature cited in the description

- **LAZAR et al.** *Proc. Natl. Acad. Sci. USA,* 2006, vol. 103, 4005-4010
- **BREZICKA et al.** *Cancer Immunol. Immunother.,* 2000, vol. 49, 235-242
- **PAGET et al.** *Arthritis Rheum.,* 1978, vol. 21, 249-255
- **BRUNNER et al.** *Immunology,* 1968, vol. 14, 181
- **WARD et al.** *Nature,* 1989, vol. 341, 544-546
- **HOLT et al.** *Trends Biotechnol,* November 2003, vol. 21 (11), 484-90
- **REVETS et al.** *Expert Opin Biol Ther.,* January 2005, vol. 5 (1), 111-24
- **BIRD et al.** *Science,* 1988, vol. 242, 423-426
- **HUSTON et al.** *PNAS USA,* 1988, vol. 85, 5879-5883
- *PNAS USA,* 1993, vol. 90 (14), 6444-8
- **ADIE et al.** *Biotechniques,* 2002, vol. 33, 1152
- **BELETSKII et al.** *Biotechniques,* 2005, vol. 39, 894
- **SOVENYHAZY et al.** *Nucl. Acids Res.,* 2003, vol. 31, 2561